# EUROPEAN PATENT SPECIFICATION

(11) **EP 1 232 143 B1**
(45) Date of publication and mention of the grant of the patent: **17.08.2005**
(21) Application number: 00979191.4
(22) Date of filing: 16.11.2000
(51) Int. Cl.: C07C 311/09, C07C 303/40

(54) **METHOD FOR PREPARING DIMETAL SULFONYL AMIDE SALTS**
VERFAHREN ZUR HERSTELLUNG VON DIMETALL-SULFONAMID-SALZEN
PROCEDE DE PREPARATION DE SELS SULFONAMIDES DIMETALLIQUES

(30) Priority: 23.11.1999 US 167020 P
(43) Date of publication of application: 21.08.2002
(73) Proprietor: E.I. DU PONT DE NEMOURS AND COMPANY, Wilmington, Delaware 19898 (US)
(72) Inventor: BLAU, Hanne, Anna, Katharina, Wilmington, DE 19806 (US)
(74) Representative: Carpmaels & Ransford
(86) International application number: PCT/US2000/031597
(87) International publication number: WO 2001/038300

(56) References cited:
- EP-A- 0 021 003
- US-A- 3 617 190
- US-A- 3 923 810
- DATABASE CHEMABS [Online] CHEMICAL ABSTRACTS SERVICE, COLUMBUS, OHIO, US; STN, CAPLUS accession no. 1984:490525, XP002161162 & V.F. KONEV ET AL: FARM. ZH. (KIEV), no. 2, 1984, pages 65-56,

## Description

### FIELD OF THE INVENTION

The present invention is directed to a high yield process for producing metal sulfonyl amide salts according to claim 1 which are highly useful as imidizing agents in the preparation of sulfonyl imides, which are in turn useful as strong acid catalysts, as electrolyte salts in electrochemical cells, and as monomers suitable for incorporation into ionomers. The products disclosed are useful in electrochemical applications such as batteries, fuel cells, electrolysis cells, ion exchange membranes, sensors and electrochemical capacitors.

### BACKGROUND OF THE INVENTION

Methods for imidizing chemical compositions containing sulfonyl fluorides particularly fluorinated sulfonyl fluorides are known in the art. For example, DesMarteau, U.S. Patent No. 5,463,005, discloses substituted perfluoro-olefins of the formula 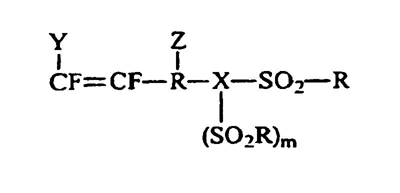 where X = CH or N, Z = H, K, Na, or Group I or 11 metal, R = one or more fluorocarbon groups including fluorocarbon ethers and/or sulfonyl groups and/or perfluoro non-oxy acid groups, Y = perfluoroalkyl or F, and m = 0 or 1.

Xue, Ph.D. thesis, Clemson University, 1996, discloses the formation of the monomer

CF₂=CF-OCF₂CF₂SO₂N(Na)SO₂CF₃

by reaction of CF₂=CF-OCF₂CF₂SO₂Cl with CF₃SO₂NHNa in the presence of Na₂CO₃ in acetonitrile.

Further disclosed by Xue, *op.cit,*, is CF₃SO₂NNa₂ made by reacting a mixture of CF₃SO₂NHNa and NaH in THF for four hours at room temperature. Xue's CF₃SO₂NNa₂ composition is reacted with a cyclic sulfone of the formula 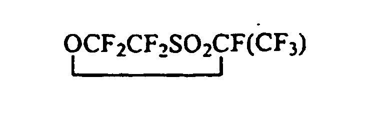 to produce the vinyl ether monomer, CF₂=CF-OCF₂CF₂SO₂N(Na)SO₂CF₃ at a yield of 4%, the remainder of the product being two saturated species, both present in larger quantities.

Behrend and Haas, J. Fluorine Chem. 4 (1974) 99-106, disclose the synthesis of CF₃SO₂NAg₂ made from CF₃SO₂NAg₂•NH₃ at 200°C. CF₃SO₂NAg₂•NH₃ is formed from CF₃SO₂NH₂ and AgNO₃ in aqueous solution while adding an aqueous solution of ammonia dropwise.

Meußdoerffer et al, Chemiker Zeitung, 96. Jahrgang (1972) No. 10, 582-583 disclose a method for synthesizing RSO₂NH₂ wherein R is perfluoroalkyl.

### SUMMARY OF THE INVENTION

The present invention provides for a process for forming a metal sulfonyl amide salt as defined in claim 1.

As used herein, the term "reacting" is intended to mean allowing at least two components in a reaction mixture to react to form at least one product. "Reacting" may optionally include stirring and/or heating or cooling.

### BRIEF DESCRIPTION OF THE DRAWING

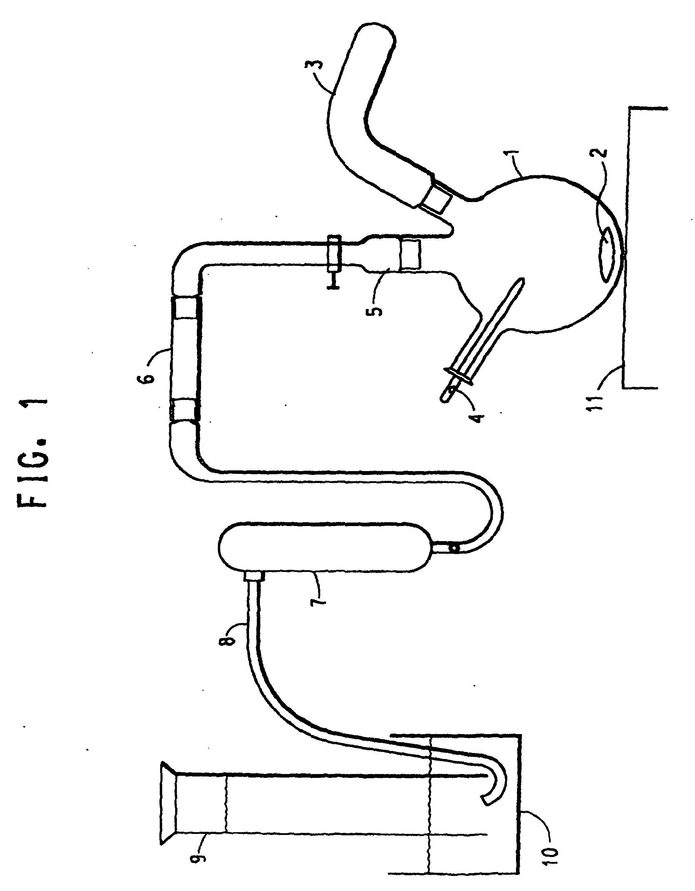

Figure 1 describes the apparatus employed for determining the amount of hydrogen evolved in the process of the invention.

### DETAILED DESCRIPTION

The metal sulfonyl amide salt (RSO₂NM_{b})_{3-b}M'_{c}, (I) composition has surprisingly high efficacy in the preparation of sulfonyl imides including unsaturated and saturated fluorosulfonylimides, and including ionomers having pendant groups comprising fluorosulfonyl imides, which are highly suitable for use in electrochemical cells. In particular, the lithium imide may serve as an electrolyte in lithium and lithium ion batteries. Until the present invention, however, (I), as CF₃SO₂NNa₂, the preferred embodiment, was available according to the process of Xue as described in the background, which consists of combining CF₃SO₂NHNa made according to known art with NaH in THF solvent at room temperature, and mixing for four hours. The product of Xue when combined with a cyclic sulfone of the formula 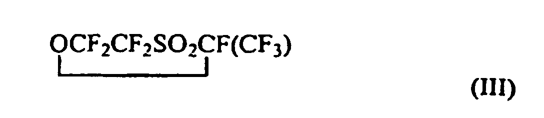 provides a mixture of fluorosulfonyl imides, by far the most useful of which is the unsaturated compound of the formula

CF₂=CFOCF₂CF₂SO₂N(Na)SO₂CF₃ (IV)

but which is prepared according to Xue in only 4% yield, an amount too small for most or commercial enterprise. The unsaturated monomer (IV) is of considerable potential commercial value for incorporation into ionomers with application in electrochemical end uses. The cyclic sulfone (III) is readily available by reactions taught in the art. There is considerable incentive to find ways to prepare the monomer (IV) from the cyclic sulfone (III).

In the process according to Xue, CF₃SO₂NHNa is combined with NaH in tetrahydrofuran (THF) for four hours at room temperature to form CF₃SO₂NNa₂. The inventor hereof has followed the teachings of Xue, and determined by ordinary analytical methods that Xue's process produces CF₃SO₂NNa₂ with conversion of less than 10%, most of the remainder of the reaction product being unconverted starting material.

The inventor hereof has found surprisingly that the metal sulfonyl amide salt (I) can be made at much higher purities than in Xue's process, purity of greater than 50%, preferably greater than 90%, most preferably greater than 95%, by contacting the sulfonyl amide or a metal salt thereof (II) with at least one alkali or alkaline earth metal hydride and an aprotic liquid according to claim 1, the reaction mixture is permitted to react to any desired degree of conversion from 50% up to 100%, which is preferred. In the sulfonyl amide or metal salt thereof (II), a=1 or 2, M" is alkaline earth metal when a = 1, M" is alkali metal or hydrogen when a = 2, and R is aryl, fluoro-aryl, or XCF₂- where X is H, halogen, or a fluorinated or non-fluorinated linear or cyclic alkyl radical having 1-10 carbons, optionally substituted by one or more ether oxygens. The hydride may be a mixture of more than one alkali or alkaline earth hydrides, or a mixture of alkali and alkaline earth hydrides. If preferred, the reaction may proceed in stages with different hydrides being fed to the reaction vessel at different times.

R is preferably perfluoroalkyl, most preferably trifluoromethyl, and the hydride is preferably sodium hydride. CF₃SO₂NH₂ is the preferred starting material. Preferably, the reaction to produce the CF₃SO₂NNa₂ is continued until one or the other starting material is completely consumed and the reaction stops. More preferably the stoichiometry is adjusted so that only trace amounts of either starting material remain when reaction is complete. The hydride is preferably added at slightly below stoichiometric quantity.

Sulfonyl amide and metal salt thereof (II), are soluble in the aprotic solvents employed in the process of preparing the metal sulfonyl amide salt (I), but the metal sulfonyl amide salt (I) itself is not. The solubility difference is exploited to separate the reaction product from the reaction mixture and obtain a composition comprising sulfonyl amide salts at least 50 mol%, preferably at least 90 mol%, most preferably at least 95 mol%, of which salts are metal sulfonyl amide salts represented by the formula (RSO₂NM_{b})_{3-b}M'_{c}, (I), as hereinabove defined. Any convenient method known in the art for separating solids from liquids may be employed, including filtration, centrifugation and, distillation.

While it is preferred to permit the reaction to run to completion, this may not always be practical depending upon the aprotic solvent chosen. In neat acetonitrile, 100% conversion is achieved in about 4 hours at room temperature. The method of separation based upon the solubility difference hereinabove described provides a practical method for isolating the metal sulfonyl amide salt (I) at high purity when conversion has been low.

It has been found that residual hydride left over from the synthesis of the metal sulfonyl amide salt (I) is not highly deleterious to the use of (I) in preparing imides from sulfonyl fluorides or cyclic sulfones. While not critical, the CF₃SO₂NNa₂ preferred for the process of the present invention is substantially free of contamination by NaH. This is achieved by employing slightly less than the stoichiometric amount of NaH in its preparation, thereby insuring that when the reaction achieves full conversion, the NaH will be exhausted. Any excess of the soluble reaction intermediate, CF₃SO₂NHNₐ, is easily separated by washing/filtration cycles, preferably using fresh aliquots of solvent.

In preparing the metal sulfonyl amide salt (I) it has been found that the components of the reaction mixture may be combined in any order, but that it is preferred to first mix the sulfonyl amide or a metal salt thereof (II), with the aprotic liquid to form a solution, followed by adding in the hydride to form a slurry after the solution has formed. Mixing the hydride with the aprotic solvent as a first step has resulted in poor reaction or slower than expected conversion.

A suitable temperature for preparing the metal sulfonyl amide salt (I) will lie between the melting point and the boiling point of the aprotic liquid selected. It has been found to be satisfactory for the practice of the invention to conduct the process of the invention at room temperature. However, somewhat higher temperatures result in faster reaction. In the most preferred embodiment of the invention, acetonitrile is employed as a solvent at a temperature between 0°C and 80°C, preferably between room temperature and 80°C, most preferably between room temperature and 60°C.

Aprotic solvents suitable for preparing the metal sulfonyl amide salt (I) is substantially free of water. Excess water causes the reaction to reverse, for example to form CF₃SO₂NHNa and NaOH, and provides a route for making a sulfonate instead of an imide. In a preferred embodiment, it has been found satisfactory to employ acetonitrile having water content less than or equal to 500 PPM, with water content less than or equal to 50 PPM more preferred. Acetonitrile is quite hygroscopic, and care should be taken in handling to avoid water contamination from the atmosphere.

The atmosphere to which the metal sulfonyl amide salt (I) is exposed should also be substantially free of water. Water vapor concentrations of 25 ppm have been found to be highly suitable. Higher levels of water vapor concentration can be tolerated, but it should be understood that the higher the water vapor concentration of the atmosphere, the greater the contamination during subsequent reaction. As a general rule, the less water the better, in whatever form.

The term "inert atmosphere", as used herein, refers to an anhydrous atmosphere having a water vapor concentration of less than about 50 ppm. It is not meant to imply a non-oxidative atmosphere. Thus, the reactions herein may be accomplished in desiccated air as well as in dry nitrogen or other non-chemically active gases. Dry nitrogen gas, however, is preferred.

In a preferred embodiment, CF₃SO₂NH₂ is dissolved at a concentration in the range of 5-10% by weight in acetonitrile in an inert atmosphere such as dry nitrogen. At higher concentrations good mixing may become more difficult to maintain as the insoluble CF₃SO₂NNa₂ product begins to form, which may create a dispersion. Therefore, at concentrations higher than 10% by weight, other forms of agitation may be preferred over simple stirring, such as ultrasonic agitation, or microfluidization such as may be achieved using a MicroFluidizer™ available from Microfluidics, Inc., Newton, MA.

While the inert atmosphere is maintained NaH is added with agitation continued until the reaction is complete in about four hours. Hydrogen gas evolution rate, determined by any convenient method known in the art, has been found to be an effective indicator of reaction. The cessation of hydrogen gas flow signals completion of the reaction. An apparatus suitable for determining hydrogen gas evolution rate is shown in Figure 1, and described hereinbelow.

The amount of NaH added depends upon the particular requirements and intentions of the practitioner hereof. Adding a slight excess over the stoichiometric amount of NaH ensures complete conversion of the CF₃SO₂NH₂ or in the alternative CF₃SO₂NHNa to CF₃SO₂NNa₂. However, this leaves the CF₃SO₂NNa₂ so prepared still contaminated with insoluble NaH from which it is difficult to separate although the residual NaH does not appear to cause problems in the intended uses for the CF₃SO₂NNa₂. On the other hand, if the goal is to achieve the cleanest possible CF₃SO₂NNa₂ then a slight deficit of NaH, below the stoichiometric amount, may be employed to ensure that the NaH will be fully consumed. Employing a deficit of NaH will result in less than complete conversion of the CF₃SO₂NH₂ or CF₃SO₂NHNa to CF₃SO₂NNa₂. The soluble residual CF₃SO₂NH₂ or CF₃SO₂NHNa is easily washed away from the insoluble CF₃SO₂NNa₂.

The metal sulfonyl amide salt (I) may be dried under vacuum at elevated temperature. A suitable temperature depends upon the specific composition thereof. The preferred CF₃SO₂NNa₂ should be dried at a temperature preferably no higher than 80°C, most preferably no higher than 65°C. Certain of the compositions of the invention, including the preferred CF₃SO₂NNa₂, have been observed to undergo decomposition aggressively when heated to the decomposition threshold. The compound is moisture sensitive and should be handled under anhydrous conditions. There is a strong possibility of spontaneous and violent decomposition of this material. Spontaneous decomposition at room temperature has been observed. It is highly recommended to never handle this material in a dry state.

The process of the invention is believed to proceed according to the following scheme: one mole of the starting sulfonyl amide (II) is combined with two moles of hydride for complete conversion to one mole of the metal sulfonyl amide salt. The reaction proceeds in a first step to effect conversion of the amide (II) first to the metal salt thereof (II) followed by additional reaction to form the metal sulfonyl amide salt (I). Thus the metal salt (II) is an intermediate formed when the amide (II) is employed as starting material.

The metal salt (II) can also be made in a separate process according to the known methods of the art, and then employed as starting material in the process of the invention.

While residual hydride is not thought to be deleterious to the efficacy of the metal sulfonyl amide salt, (I) product in its intended uses, residual monometal amide/salt is not inert and interferes considerably; an exception is the case wherein R is a phenyl group. In certain cases of imidizing sulfonyl fluoride containing compounds having highly sensitive unsaturation, it is found that PhSO₂NHNa acts in a manner similar to that of the PhSO₂NNa₂ of the invention in that it does not attack the double bond.

The aprotic solvent of the process of the invention comprises acetonitrile. Acetonitrile has been found to accelerate the conversion by a considerable amount over other aprotic solvents. In neat acetonitrile, essentially quantitative conversion is achieved in about 4 hours. In the presence of as little as 5% acetonitrile in THF essentially quantitative conversion is achieved in about 25 h. These results contrast starkly with the six days required under the conditions taught by Xue.

It is found in the practice of the invention that solvent selection effects the rate of conversion, though most aprotic solvents will lead to high conversion over sufficient time. Acetonitrile is highly preferred. Other aliphatic and aromatic nitriles do not appear to be particularly better than the THF employed by Xue but may be employed as substitutes for THF. Aprotic nitriles include higher alkyl nitriles, dinitriles such as adiponitrile, benzonitrile, and the like. Other aprotic solvents include ethers, dimethyl formamide (DMF), dimethylsulfoxide (DMSO), dimethul acetamide (DMAC), and amides. Combinations of solvents are also suitable for the practice of the invention.

A highly purified form of the metal sulfonyl amide salt (RSO₂NM_{b})_{3-b}M', (I), greater than 95% purity, is readily achieved in the process of the invention. This purified form is then suitable to perform the various imidization reactions hereinbelow exemplified producing pure product at high yields. The purity of the imidized product depends directly upon the purity of (I) prepared according to the invention. Any of the methods of preparation herein described are capable of providing (I) in purities of greater than 95%.

In the process of the present invention an alkali or alkaline earth hydride is contacted with an aprotic solvent and a sulfonyl amide or monometal salt thereof (RSO₂NH)₃₋ₐM" thereby forming a reaction mixture wherein a=1 or 2, M" is alkaline earth metal when a = 1, M" is alkali metal or hydrogen when a = 2, and R is aryl, fluoro-aryl, or XCF₂- where X is H, halogen, or a fluorinated or non-fluorinated linear or cyclic alkyl radical having 1-10 carbons, optionally substituted by one or more ether oxygens. Preferably the hydride is sodium hydride, M" is hydrogen, and R is perfluoroalkyl having 1-4 carbons. Most preferably, R is CF₃-. In this process, the larger R is, the slower the reaction and the lower the conversion rate.

The composition above mentioned may include a mixture of metals forming the amide salt, including a mixture of alkali and alkaline earth metals in the same molecule. Thus, for example, if two moles of CF₃SO₂NH₂ is combined with one mole of CaH₂ followed by two moles of NaH, the resulting product will comprise (CF₃SO₂NNa)₂Ca. Many other mixtures of metals may be obtained in the current process and are encompassed therewithin.

In one embodiment of the practice of the invention, the reaction mixture is agitated, at a temperature between the freezing point and boiling point of the solvent. Room temperature has been found to be satisfactory in the preferred embodiment. The mixture is permitted to react for as long as is required to reach a degree of conversion of at least 50%. Degree of conversion is conveniently determined by measuring the volume of hydrogen gas evolved according to any method known in the art. Preferably, the reaction is allowed to proceed until no further evolution of hydrogen gas is observed indicating complete reaction. In an embodiment in which excess NaH is employed, this point is found to correspond to the point where no fluorine signal can be detected by NMR in the solvent.

Preferably, the metal sulfonyl amide salt reaction product (I) is separated from the solvent and any residual starting material, normally by a combination of washing and filtration. It has been found that CF₃SO₂NNa₂ exhibits slight solubility in ether solvents such as THF but is quite insoluble in acetonitrile. To facilitate separation, acetonitrile is therefore further referred.

The composition mentioned above, particularly the preferred composition having a molar concentration of CF₃SO₂NNa₂ exceeding 90% with respect to the total concentration of sulfonyl amide salt, is surprisingly well-suited to forming imides from sulfonyl fluoride containing fluorinated compounds, as well as for producing a highly desirable fluoromonomer from a fluorocyclic sulfone. The CF₃SO₂NNa₂ composition can convert a wide variety of unsaturated compounds having sulfonyl fluoride functionality to the corresponding sodium imide without the need to protect the double bond. Thus, a polymerizable ionic monomer can be prepared therefrom in a single-step reaction. The ionic monomers are of particular utility in forming copolymers with vinylidene fluoride, followed by ion exchange to the lithium imide form, to form a solid polymer electrolyte particularly well-suited for use in lithium and lithium ion batteries.

In like manner, the preferred CF₃SO₂NNa₂ composition is also useful in converting polymers having pendant sulfonyl fluoride functionality to the corresponding sodium imides. In a particularly surprising aspect, polymers comprising partially fluorinated monomer units, most particularly, having vinylidene fluoride monomer units in combination with fluorovinylether sulfonyl fluoride monomer units, can be converted to the corresponding imide without attack on the highly reactive backbone. This represents the only method for performing this reaction. No known method of imidization of sulfonyl fluorides can be performed on polymers having vinylidene fluoride monomer units without attacking the backbone of the polymer.

The process of the present invention as herein described is highly effective at producing the (RSO₂NMₐ)₃₋ₐM' product at high conversions and high purities at conversion rates which are suitable for commercial purposes. The invention is further illustrated in the following specific embodiments.

### EXAMPLES

### EXAMPLE 1

CF₃SO₂NH₂was purchased from Tokyo Chemical Industry, Portland, Oregon, (TCI) and dried and purified by two cycles of sublimation under a vacuum of about 0.1 Pa (10⁻³ Torr), employing a water cooled (~20°C) cold-finger, and an oil bath at 80°C. Anhydrous acetonitrile was purchased from EM Science Gibbstown, New Jersey, slurried with P₂O₅ and distilled to ensure dryness, and stored over molecular sieves inside a dry box until ready to be used. Sodium hydride (95%) was purchased from Aldrich Chemical.

Inside a model HE-63-P dry-box (Vacuum Atmosphere Company. Hawthorne, California) having a dry nitrogen atmosphere, a round bottom flask was charged with 30.003 g of the sublimed CF₃SO₂NH₂ and 750 ml of the dried acetonitrile. 9.003 g of the sodium hydride was slowly added over a period of 60 min while the reaction mixture was stirred with a magnetic stir bar. The temperature of the reaction mixture increased from 21.6°C to 50.5°C during the addition process. The mixture was stirred at room temperature for 20 h. After 4-5 hours the reaction medium had taken on an opaque "creamy" appearance, and no further bubbling, indicative of the evolution of hydrogen, was observed.

The reacted mixture was filtered through a glass-filter (medium porosity) inside the dry-box. The white solid was washed three times with 100 ml of the anhydrous acetonitrile, transferred from the filter to a Schlenk flask and dried under vacuum of 1 Pa (10⁻² Torr) at room temperature for 5 h., still in the dry box. Approximately 10% of the filtrate was lost in transferring from the filter to the Schlenk flask. The Schlenk flask was sealed, removed from the dry-box, and subject to further evacuation under oil pump vacuum 0.1 Pa (10⁻³ Torr) for 15 h at room temperature. The Schlenk flask was then immersed in an oil bath set at 50°C and held for four hours at which time the bath was heated to 65 °C and the Schlenk flask was held therein for an additional 20 h while still subject to evacuation under oil pump vacuum 0.1 Pa (10⁻³ Torr). Afterwards, the CF₃SO₂NNa₂ was only handled inside the dry-box.

30.0 grams of product were isolated. The product decomposed at 110°C while generating large amounts of a gas.

### EXAMPLE 2

Inside the dry box of Example 1, a flask was charged with 5.142 g C₄F₉SO₂NH₂ made from C₄F₉SO₂F and NH₃ according to the method of Meußdoerffer et al, *op.cit*., and 100 ml of anhydrous acetonitrile prepared as in Example 1. 0.784 g NaH (Aldrich) was slowly added over a period of 5 min. The mixture was stirred at room temperature for 24 h without observation. Insoluble C₄F₉SO₂NNa₂ had precipitated at the bottom of the flask. The reaction mixture was filtered through a glass filter (fine porosity) and the white residue was washed three times with 50 ml of anhydrous acetonitrile. The residue was collected from the filter and placed in a Schlenk-flask. Afterwards, the material was brought outside the dry-box and dried under oil pump vacuum 0.1 Pa (10⁻³ Torr) for 24 h at an oil bath temperature of 65°C. C₄F₉SO₂NNa₂ was only handled inside the dry-box. 4.37 g of product were isolated.

### EXAMPLE 3

In this Example, an apparatus was employed for determining the volume of hydrogen gas evolved by the reaction as a function of time. The apparatus is depicted in Figure **1.** One neck of a three necked round bottom flask, **1,** holding a magnetic stirring bar, **2,** was fitted with a solid reactant addition device SRAD, **3,** having a 75° angle to be employed for feeding a solid to the flask. A second neck was fitted with a thermocouple probe, **4,** and a third neck was fitted with a stopcock, **5.** The stopcock, **5,** was connected via a **4**cm piece of Tygon® tubing, **6,** to an Aldrich Safe-purge (TM) valve, **7,** containing mineral oil. The Safe-purge valve, **7,** was connected via a rubber hose, **8,** to a water-filled 250 ml graduated cylinder, **9,** that is deployed upside-down in a water-filled 600 ml beaker, **10.** The flask, **1**, was positioned on a magnetic stirrer, **11.** In operation liquid reactants were charged to the flask through any of the necks,SRAD, **3,** was charged with the desired amount of solid reactant and reaffixed to the flask, **1**, in the downward-pointing position shown in the figure. The beaker, **10**, was filled to about 50% of capacity with water while the graduated cylinder, **9,** was filled completely with water. The stopcock, **5,** was opened, and the adaptor, **3,** was inverted thus delivering the solid reactant to the reactants in the flask and thereby initiating the reaction. As hydrogen was evolved from the reaction it displaces the water from the graduated cylinder providing a volumetric means for determining the rate and total amount of hydrogen evolution.

Employing the methods and material of Example 1, inside the dry-box, 0.546 g of the sublimed CF₃SO₂NH₂ was dissolved in 100 ml of the anhydrous acetonitrile in the three neck round bottom flask of Figure 1. 0.213 g of the sodium hydride was carefully placed in the SRAD. The closed flask was carefully brought outside the dry box and conncected to the remainder of the apparatus of Figure 1. After all connections had been established, the stopcock to the reaction flask was opened. The reaction mixture was stirred at room temperature and the SRAD was inverted thereby feeding the NaH to the solution in the flask. Immediately, a reaction could be observed. 80 ml of gas were collected over a period of 5 min. The temperature of the reaction mixture increased from 23°C to 26°C. Over the next 120 min, the formation of gas slowed down and 74 ml of gas were collected in the graduated cylinder. During this period, the appearance of the reaction mixture changed. The fine residue in the reaction mixture changed to a thicker precipitate that settled easily to the bottom of the flask when the stirring was stopped. The reaction mixture was stirred for another hour at room temperature, 10 ml of additional gas were collected during this period. The flask was brought into the dry box and a sample of the solution was submitted for NMR. No fluorine could be detected, indicating the complete conversion of CF₃SO₂NHNa into insoluble CF₃SO₂NNa₂.

### EXAMPLE 4

Excess CF₃SO₂NH₂ and NaOH were reacted in water to prepare CF₃SO₂NNaH. Water and excess CF₃SO₂NH₂ were removed under vacuum of 0.1 Pa (10⁻³ Torr) at 70°C; the residue was dried for 16 h at 0.1 Pa (10⁻³ Torr) at 110°C. Following the procedures of Example 1, inside the dry box, a 250 ml two neck round bottom flask with a magnetic stirring bar was charged with 1.034 g of the CF₃SO₂NNaH. The material was dissolved in 100 ml of anhydrous acetonitrile of Example 1. The procedures of Example 3 were followed but the three necked flask was replaced by the two-necked flask and the thermocouple was omitted. The reaction mixture was stirred at room temperature and the SRAD was inverted thereby feeding the NaH to the solution in the flask. No immediate reaction could be observed. Over the first 150 min, only a total of 10 ml of an evolving gas could be collected. After 150 min, the formation of gas started. Over the next 105 min, additional 135 ml of gas were collected in the graduated cylinder. During this period, the appearance of the reaction mixture changed. The fine residue in the reaction mixture changed to a thicker precipitation that settled easily at the bottom of the flask when the stirring was stopped. The reaction mixture was stirred for another 14 h at room temperature. 10 ml of additional gas were collected during this period. The flask was brought into the dry box and a sample of the solution was submitted for NMR. No fluorine could be detected, indicating the complete conversion of CF₃SO₂NHNa into insoluble CF₃SO₂NNa₂.

### EXAMPLE 5

Following the procedure of Example 4, the flask was charged with 0.939 g of the CF₃SO₂NHNa which was dissolved in 100 ml anhydrous acetonitrile. 0.214 g of the NaH were placed in the SRAD. After connection to the remainder of the apparatus of Figure 1, the reaction mixture was stirred at room temperature and the SRAD was inverted thereby feeding the NaH to the solution in the flask. No immediate reaction could be observed. Over the first 3 h, only a total of 5 ml of gas was evolved. Over the next 60 min, the formation of gas was observed and 135 ml of gas were collected in the graduated cylinder. During this period, the appearance of the reaction mixture changed. The fine residue in the reaction mixture changed to a thicker precipitate that settled easily at the bottom of the flask when the stirring was stopped. The gas evolution rate was observed to decrease greatly after about 4 hours of reaction. The reaction mixture was stirred for another 14 h at room temperature. 20 ml of additional gas were collected during this period. The flask was brought into the dry box and a sample of the solution was submitted for NMR. No fluorine could be detected, indicating the complete conversion of CF₃SO₂NHNa into insoluble CF₃SO₂NNa₂.

### EXAMPLE 6

Following the procedure of Example 11, a 250 ml two neck round bottom flask was charged with 0.189 g NaH and 50 ml of anhydrous acetonitrile prepared as in Example 1. 0.879 g of the CF₃SO₂NHNa prepared in Example 4 was dissolved in 50 ml of anhydrous acetonitrile prepared as in Example 1 and placed in an addition funnel which replaced the SRAD device of Example 4. After making the required connections, the reaction mixture was stirred at room temperature for 4 h. No gas formation was observed. The CF₃SO₂NHNa solution was added and the reaction mixture was continued to be stirred at room temperature. After 18 h, only a total of 20 ml of an evolving gas could be collected. The reaction mixture was bright yellow and did not look like expected. A lot of a yellow precipitate was formed. The reaction mixture was discarded.

The reason why this reaction did not proceed as expected is not known.

### COMPARATIVE EXAMPLE 1

Inside the dry-box of Example 1, a flask was charged with 0.93 g of CF₃SO₂NHNa from Example 11, 0.135 g NaH (Aldrich) and 20 ml of anhydrous THF (Aldrich; distilled off Na metal). The reaction mixture was stirred for 4 h at room temperature and was then filtered through a glass filter (fine porosity). The filtrate was collected in a flask and brought outside the dry-box. All solvents were removed under vacuum 0.1 Pa (10⁻³ Torr) and the residue was heated to 65 °C for 24 h at 0.1 Pa (10⁻³ Torr). 0.862 g (5.04 mmol) of CF₃SO₂NHNa were recovered, corresponding to 92.6 % of the starting material. The dried material was brought into the dry-box and 50 ml of anhydrous acetonitrile were added because it is suspected that CF₃SO₂NNa₂ is slightly soluble in THF. The majority of the material was dissolved in the acetonitrile and only a slight trace of a solid could be observed in the solution. It was not attempted to separate this residue. It should be safe to assume that less than 10% of the CF₃SO₂NHNa have been converted to CF₃SO₂NNa₂ after 4 h in THF at room temperature.

## Claims

1. A process for forming a sulfonyl amide salt represented by the formula (RSO₂NM_{b})_{3-b}M'_{c} wherein R is aryl, fluoro-aryl, or XCF₂- where X is H, halogen, fluorinated or non-fluorinated linear or cyclic alkyl radicals having 1-10 carbons, optionally substituted by one or more ether oxygens, M' is an alkaline earth metal, b=1 or 2, c=0 or 1, M is alkaline earth or alkali metal when b is 1 or 2 respectively and c=0, and M is alkali metal when b=1 and c=1, with the proviso that c≠1 when b=2, the process comprising combining in an atmosphere having a water vapor concentration of less than 50 parts per million
at least one alkali or alkaline earth hydride,
a sulfonyl amide or monometal sulfonyl amide salt thereof having the formula
(RSO₂NH)₃₋ₐM" (II)
wherein a=1 or 2, M" is alkaline earth metal when a = 1, M" is alkali metal or hydrogen when a = 2, and R is aryl, fluoro-aryl, or XCF₂- where X is H, halogen, or a fluorinated or non-fluorinated linear or cyclic alkyl radical having 1-10 carbons, optionally substituted by one or more ether oxygens;
and,
at least one aprotic liquid substantially free of water,
thereby forming a reaction mixture; and,
reacting said reaction mixture forming a precipitate of (RSO₂NM_{b})_{3-b}M'_{c}; and
separating said precipitate from said liquid, wherein said aprotic liquid comprises acetonitrile.

2. The process of Claim 1 wherein the hydride is sodium hydride.

3. The process of Claim 1 wherein R is perfluoroalkyl.

4. The process of Claim 3 wherein R is trifluoromethyl.

5. The process of Claim 1 further comprising the step of heating the mixture to a temperature in the range of room temperature to 60°C.

6. The process of Claim 1 wherein the conversion is at least 90 mol-%.

7. The process of Claim 1 wherein M" is H.

8. 15. The process of Claim 1 wherein the aprotic liquid and the (RSO₂NH)₃₋ₐM" are mixed to form a solution at the same time as or prior to the addition of the hydride.

## Patentansprüche

1. Verfahren zum Erzeugen eines Sulfonamid-Salzes, das dargestellt wird durch die Formel (RSO₂NM_{b})_{3-b}M'_{c}, worin R Aryl ist, Fluoraryl oder XCF₂-, worin X H ist, Halogen, fluorierte oder nichtfluorierte, geradkettige oder cyclische Akyl-Reste mit 1 bis 10 Kohlenstoffatomen, wahlweise substituiert durch ein oder mehrere Ether-Sauerstoffatome, M' ist ein Erdalkalimetall, b = 1 oder b = 2, c ist Null oder 1, M ist ein Erdalkalimetall oder ein Alkalimetall, wenn b 1 bzw. 2 ist und c = 0 ist, und M ist ein Alkalimetall, wenn b = 1 und c = 1 gelten unter der Voraussetzung, dass c ≠ 1 ist, wenn b = 2 ist;
welches Verfahren in einer Atmosphäre mit einer Wasserdampfkonzentration von 50 Teilen pro 1 Million das Vereinen umfasst von:
mindestens einem Alkali- oder Erdalkalimetallhydrid,
einem Sulfonamid oder Monometallsulfonamid-Salz davon, das die Formel hat:
(RSO₂NH)₃₋ₐM" (II)
worin a = 1 oder 2 gilt und M" ein Erdalkalimetall ist, wenn a = 1 ist und M" ein Alkalimetall oder Wasserstoff ist, wenn a = 2 ist, und R ist Aryl, Fluoraryl oder XCF₂-, worin X H ist, Halogen, fluorierte oder ein nichtfluorierter, geradkettiger oder cyclischer Akyl-Rest mit 1 bis 10 Kohlenstoffatomen, wahlweise substituiert durch ein oder mehrere Ether-Sauerstoffatome;
und
mindestens einer aprotischen Flüssigkeit, die weitgehend frei ist von Wasser,
wodurch ein Reaktionsgemisch erzeugt wird; und
Umsetzen des Reaktionsgemisches unter Erzeugung eines Niederschlags aus (RSO₂NM_{b})_{3-b}M'_{c}; sowie
Abtrennen des Niederschlags von dieser Flüssigkeit, wobei diese aprotische Flüssigkeit Acetonitril aufweist.

2. Verfahren nach Anspruch 1, wobei das Hydrid Natriumhydrid ist.

3. Verfahren nach Anspruch 1, wobei R Perfluoralkyl ist.

4. Verfahren nach Anspruch 3, wobei R Trifluormethyl ist.

5. Verfahren nach Anspruch 1, ferner umfassend den Schritt des Erhitzens der Mischung bis zu einer Temperatur in einem Bereich von Raumtemperatur bis 60°C.

6. Verfahren nach Anspruch 1, wobei die Umwandlung mindestens 90 Molprozent beträgt.

7. Verfahren nach Anspruch 1, wobei M" H ist.

8. Verfahren nach Anspruch 1, wobei die aprotische Flüssigkeit und das (RSO₂NH)₃₋ₐ M" zur Erzeugung einer Lösung gleichzeitig mit oder vor der Zugabe des Hydrids gemischt werden.

## Revendications

1. Procédé pour la formation d'un sel de sulfonylamide représenté par la formule (RSO₂NM_{b})_{3-b}M'_{c}, dans laquelle R est un radical aryle, fluoroaryle ou XCF₂- où X est H, un halogène, des radicaux alkyles linéaires ou cycliques fluorés ou non fluorés possédant 1-10 carbones, éventuellement substitués par un ou plusieurs oxygènes d'éther, M' est un métal alcalino-terreux, b = 1 ou 2, c = 0 ou 1, M est un métal alcalino-terreux ou alcalin lorsque b est 1 ou 2 respectivement et c = 0, et M est un métal alcalin lorsque b = 1 et c = 1, sous réserve que c ≠ 1 lorsque b = 2,
le procédé comprenant la combinaison dans une atmosphère avec une concentration de vapeur d'eau de moins de 50 parties par million:
d'au moins un hydrure alcalin ou alcalino-terreux,
d'un sulfonylamide ou un sel de sulfonylamide monométal de celui-ci présentant la formule:
(RSO₂NH)₃₋ₐM" (II)
dans laquelle a = 1 ou 2, M" est un métal alcalino-terreux lorsque a = 1, M" est un métal alcalin ou un hydrogène lorsque a = 2, et R est un radical aryle, fluoroaryle ou XCF₂- où X est H, un halogène, des radicaux alkyles linéaires ou cycliques fluorés ou non fluorés possédant 1-10 carbones, éventuellement substitués par un ou plusieurs oxygènes d'éther;
et,
d'au moins un liquide aprotique substantiellement exempt d'eau,
formant ainsi un mélange réactionnel; et,
la réaction dudit mélange réactionnel formant un précipité de (RSO₂NM_{b})_{3-b}M'_{c}; et
la séparation dudit précipité à partir dudit liquide,
dans lequel ledit liquide aprotique comprend de l'acétonitrile.

2. Procédé suivant la revendication 1, dans lequel l'hydrure est de l'hydrure de sodium.

3. Procédé suivant la revendication 1, dans lequel R est un radical perfluoroalkyle.

4. Procédé suivant la revendication 3, dans lequel R est un radical trifluorométhyle.

5. Procédé suivant la revendication 1, comprenant en outre l'étape de chauffage du mélange à une température dans l'intervalle de la température ambiante à 60°C.

6. Procédé suivant la revendication 1, dans lequel la conversion est d'au moins 90% en moles.

7. Procédé suivant la revendication 1, dans lequel M" est H.

8. Procédé suivant la revendication 1, dans lequel le liquide aprotique et (RSO₂NH)₃₋ₐM" sont mélangés pour former une solution en même temps que ou avant l'ajout de l'hydrure.
